# EUROPEAN PATENT SPECIFICATION

(11) **EP 2 521 795 B1**
(45) Date of publication and mention of the grant of the patent: **20.11.2013**
(21) Application number: 11702721.9
(22) Date of filing: 07.01.2011
(51) Int. Cl.: C12Q 1/68, C12N 9/12, C12N 9/90

(54) **MATERIALS AND METHODS FOR ISOTHERMAL NUCLEIC ACID AMPLIFICATION**
MATERIALIEN UND METHODEN ZUR ISOTHERMEN AMPLIFIKATION VON NUCLEINSÄUREN
COMPOSITIONS ET PROCÉDÉS D'AMPLIFICATION ISOTHERME D'ACIDES NUCLEIQUES

(30) Priority: 08.01.2010 US 293372 P
(43) Date of publication of application: 14.11.2012
(73) Proprietor: QIAGEN Gaithersburg, Inc., Gaithersburg, MD 21122 (US)
(72) Inventor: LOWE, Brian, Olney Maryland 20832 (US); FULBRIGHT, Anna, Columbia Maryland 21046 (US)
(74) Representative: Roth, Carla
(86) International application number: PCT/US2011/020459
(87) International publication number: WO 2011/085160

(56) References cited:
- WO-A1-2005/095654
- WO-A1-2010/088273
- WO-A2-2004/027025
- WO-A2-2006/074334
- WO-A2-2007/044671
- WO-A2-2007/120808
- US-A1- 2008 268 498
- MOTRE A ET AL: "Enhancing helicase-dependent amplification by fusing the helicase with the DNA polymerase", GENE, ELSEVIER, AMSTERDAM, NL, vol. 420, no. 1, 15 August 2008 (2008-08-15), pages 17-22, XP022820485, ISSN: 0378-1119, DOI: DOI:10.1016/J.GENE.2008.04.017 [retrieved on 2008-05-08]
- GOLDMEYER JAMES ET AL: "Development of a novel one-tube isothermal reverse transcription thermophilic helicase-dependent amplification platform for rapid RNA detection.", THE JOURNAL OF MOLECULAR DIAGNOSTICS : JMD NOV 2007 LNKD- PUBMED:17975029, vol. 9, no. 5, November 2007 (2007-11), pages 639-644, XP002628183, ISSN: 1525-1578
- YONG-JOO JEONG ET AL: "Isothermal DNA amplification in vitro: the helicase-dependent amplification system", CMLS CELLULAR AND MOLECULAR LIFE SCIENCES, BIRKHÄUSER-VERLAG, BA, vol. 66, no. 20, 24 July 2009 (2009-07-24) , pages 3325-3336, XP019755988, ISSN: 1420-9071, DOI: DOI:10.1007/S00018-009-0094-3
- AN LIXIN ET AL: "Characterization of a thermostable UvrD helicase and its participation in helicase-dependent amplification", JOURNAL OF BIOLOGICAL CHEMISTRY, AMERICAN SOCIETY FOR BIOCHEMISTRY AND MOLECULAR BIOLOGY, INC, US, vol. 280, no. 32, 13 June 2005 (2005-06-13), pages 28952-28958, XP002400121, ISSN: 0021-9258, DOI: DOI:10.1074/JBC.M503096200

### SEQUENCE LISTING

<110> Qiagen Gaithersburg, Inc.
   Lowe, Brian
<120> MATERIALS AND METHODS FOR ISOTHERMAL NUCLEIC ACID AMPLIFICATION
<130> 53 700 K
<140> 11702721.9
   <141> 2011-01-07
<150> 61/293,372
   <151> 2010-01-08
<160> 8
<170> PatentIn version 3.5
<210> 1
   <211> 7500
   <212> DNA
   <213> Chlamydia trachomatis
<400> 1
   tttgcaactc ttggtggtag actttgcaac tcttggtggt agactttgca actcttggtg      60
   gtagactttg caactcttgg tggtagactt ggtcataatg gacttttgtt gaaaaatttc     120
   ttaaaatctt agagctccga ttttgaatag ctttggttaa gaaaatgggc tcgatggctt     180
   tccataaaag taggttgttc ttaacttttg gggacgcgtc ggaaatttgg ttatctactt     240
   tatctcatct aactagaaaa aattatgcgt ctgggattaa ctttcttgtt tctttagaga     300
   ttctggattt atcggaaacc ttgataaagg ctatttctct tgaccacagc gaatctttgt     360
   ttaaaatcaa gtctctagat gtttttaatg gaaaagtcgt ttcagaggcc tctaaacagg     420
   ctagagcggc atgctacata tctttcacaa agtttttgta tagattgacc aagggatata     480
   ttaaacccgc tattccattg aaagattttg gaaacactac attttttaaa atccgagaca    540
   aaatcaaaac agaatcgatt tctaagcagg aatggacagt tttttttgaa gcgctccgga      600
   tagtgaatta tagagactat ttaatcggta aattgattgt acaagggatc cgtaagttag     660
   acgaaatttt gtctttgcgc acagacgatc tattttttgc atccaatcag atttcctttc      720
   gcattaaaaa aagacagaat aaagaaacca aaattctaat cacatttcct atcagcttaa     780
   tggaggagtt gcaaaaatac acttgtggga gaaatgggag agtatttgtt tctaaaatag     840
   ggattcctgt aacaacaagt caggttgcgc ataattttag gcttgcagag ttctatagtg     900
   ctatgaaaat aaaaattact cctagagtac ttcgtgcaag cgctttgatt catttaaagc     960
   aaataggatt aaaagatgag gaaatcatgc gtatttcctg tctttcatcg agacaaagtg    1020
   tgtgttctta ttgttctggg gaagaggtaa gtcctctagt acaaacaccc ccaatattgt    1080
   gatataatta aaattatatt catattctgt tgccagaaaa aacactttta ggctatatta    1140
   gagccatctt ctttgaagcg ttgtcttctc gagaagattt atcgtacgca aatatcatct    1200
   ttgcggttgc gtgtcctgtg accttcatta tgtcggagtc tgagcaccct aggcgtttgt    1260
   actccgtcac agcggttgct cgaagcacgt gcggggttat cttaaaaggg attgcagctt    1320
   gtagtcctgc ttgagagaac gtgcgggcga tttgccttaa ccccaccatt tttccggagc    1380
   gagttacgaa gacaaaacct cttcgttgac cgatgtactc ttgtagaaag tgcataaact    1440
   tctgaggata agttataata atcctctttt ctgtctgacg gttcttaagc tgggagaaag    1500
   aaatggtagc ttgttggaaa caaatctgac taatctccaa gcttaagact tcagaggagc    1560
   gtttacctcc ttggagcatt gtctgggcga tcaaccaatc ccgggcattg atttttttta    1620
   gctcttttag gaaggacgct gtttgcaaac tgttcatcgc atctgttttt actatttccc    1680
   tggttttaaa aaatgttcga ctattttctt gtttagaagg ttgcgctata gcgactattc    1740
   cttgagtcat cctgtttagg aatcttgtta aggaaatata gcttgctgct cgaacttgtt    1800
   tagtaccttc ggtccaagaa gtcttggcag aggaaacttt tttaatcgca tctagaatta    1860
   gattatgatt taaaagggaa aactcttgca gattcatatc caaggacaat agaccaatct    1920
   tttctaaaga caaaaaagat cctcgatatg atctacaagt atgtttgttg agtgatgcgg    1980
   tccaatgcat aataacttcg aataaggaga agcttttcat gcgtttccaa taggattctt    2040
   ggcgaatttt taaaacttcc tgataagact tttcgctata ttctaacgac atttcttgct    2100
   gcaaagataa aatcccttta cccatgaaat ccctcgtgat ataacctatc cgtaaaatgt    2160
   cctgattagt gaaataatca ggttgttaac aggatagcac gctcggtatt tttttatata    2220
   aacatgaaaa ctcgttccga aatagaaaat cgcatgcaag atatcgagta tgcgttgtta    2280
   ggtaaagctc tgatatttga agactctact gagtatattc tgaggcagct tgctaattat    2340
   gagtttaagt gttctcatca taaaaacata ttcatagtat ttaaatactt aaaagacaat    2400
   ggattaccta taactgtaga ctcggcttgg gaagagcttt tgcggcgtcg tatcaaagat    2460
   atggacaaat cgtatctcgg gttaatgttg catgatgctt tatcaaatga caagcttaga    2520
   tccgtttctc atacggtttt cctcgatgat ttgagcgtgt gtagcgctga agaaaatttg    2580
   agtaatttca ttttccgctc gtttaatgag tacaatgaaa atccattgcg tagatctccg    2640
   tttctattgc ttgagcgtat aaagggaagg cttgacagtg ctatagcaaa gactttttct    2700
   attcgcagcg ctagaggccg gtctatttat gatatattct cacagtcaga aattggagtg    2760
   ctggctcgta taaaaaaaag acgagcaacg ttctctgaga atcaaaattc tttctttgat    2820
   gccttcccaa caggatacaa ggatattgat gataaaggag ttatcttagc taaaggtaat    2880
   ttcgtgatta tagcagctag gccatctata gggaaaactg ctttagctat agacatggcg    2940
   ataaatcttg cggttactca acagcgtaga gttggtttcc tatctctaga aatgagcgca    3000
   ggtcaaattg ttgagcggat tattgctaat ttaacaggaa tatctggtga aaaattacaa    3060
   agaggggatc tctctaaaga agaattattc cgagtagaag aagctggaga aacagttaga    3120
   gaatcacatt tttatatctg cagtgatagt cagtataagc ttaatttaat cgcgaatcag    3180
   atccggttgc tgagaaaaga agatcgagta gacgtaatat ttatcgatta cttgcagttg    3240
   atcaactcat cggttggaga aaatcgtcaa aatgaaatag cagatatatc tagaacctta    3300
   agaggtttag cctcagagct aaacattcct atagtttgct tatcccaact atctagaaaa    3360
   gttgaggata gagcaaataa agttcccatg ctttcagatt tgcgagacag cggtcaaata 3420
   gagcaagacg cagatgtgat tttgtttatc aataggaagg aatcgtcttc taattgtgag 3480
   ataactgttg ggaaaaatag acatggatcg gttttctctt cggtattaca tttcgatcca 3540
   aaaattagta aattctccgc tattaaaaaa gtatggtaaa ttatagtaac tgccacttca 3600
   tcaaaagtcc tatccacctt gaaaatcaga agtttggaag aagacctggt caatctatta 3660
   agatatctcc caaattggct caaaatggga tggtagaagt tataggtctt gattttcttt 3720
   catctcatta ccatgcatta gcagctatcc aaagattgct gactgcaacg aattacaagg 3780
   ggaacacaaa aggggttgtt ttatccagag aatcaaatag ttttcaattt gaaggatgga 3840
   taccaagaat ccgttttaca aaaactgaat tcttagaggc ttatggagtt aagcggtata 3900
   aaacatccag aaataagtat gagtttagtg gaaaagaagc tgaaactgct ttagaagcct 3960
   tataccattt aggacatcaa ccgtttttaa tagtggcaac tagaactcga tggactaatg 4020
   gaacacaaat agtagaccgt taccaaactc tttctccgat cattaggatt tacgaaggat 4080
   gggaaggttt aactgacgaa gaaaatatag atatagactt aacacctttt aattcaccat 4140
   ctacacggaa acataaaggg ttcgttgtag agccatgtcc tatcttggta gatcaaatag 4200
   aatcctactt tgtaatcaag cctgcaaatg tataccaaga aataaaaatg cgcttcccaa 4260
   atgcatcaaa gtatgcttac acatttatcg actgggtgat tacagcagct gcgaaaaaga 4320
   gacgaaaatt aactaaggat aattcttggc cagaaaactt gttcttaaac gttaacgtta 4380
   aaagtcttgc atatatttta aggatgaatc ggtacatttg tacaaggaac tggaaaaaaa 4440
   tcgagttagc tatcgataaa tgtatagaaa tcgccattca gcttggttgg ttatctagaa 4500
   gaaaacgcat tgaatttctg gattcttcta aactctctaa aaaagaaatt ctatatctaa 4560
   ataaagagcg ttttgaagaa ataaccaaga aatctaaaga acaaatggaa caattagaac 4620
   aagaatctat taattaatag caaacttgaa actaaaaacc taatttattt aaagctcaaa 4680
   ataaaaaaga gttttaaaat gggaaattct ggtttttatt tgtataacac tcaaaactgc 4740
   gtctttgctg ataatatcaa agttgggcaa atgacagagc cgctcaagga ccagcaaata 4800
   atccttggga caacatcaac acctgtcgca gccaaaatga cagcttctga tggaatatct 4860
   ttaacagtct ccaataatcc atcaaccaat gcttctatta caattggttt ggatgcggaa 4920
   aaagcttacc agcttattct agaaaagttg ggagatcaaa ttcttggtgg aattgctgat 4980
   actattgttg atagtacagt ccaagatatt ttagacaaaa tcacaacaga cccttctcta 5040
   ggtttgttga aagcttttaa caactttcca atcactaata aaattcaatg caacgggtta 5100
   ttcactccca ggaacattga aactttatta ggaggaactg aaataggaaa attcacagtc 5160
   acacccaaaa gctctgggag catgttctta gtctcagcag atattattgc atcaagaatg 5220
   gaaggcggcg ttgttctagc tttggtacga gaaggtgatt ctaagcccta cgcgattagt 5280
   tatggatact catcaggcgt tcctaattta tgtagtctaa gaaccagaat tattaataca 5340
   ggattgactc cgacaacgta ttcattacgt gtaggcggtt tagaaagcgg tgtggtatgg 5400
   gttaatgccc tttctaatgg caatgatatt ttaggaataa caaatacttc taatgtatct 5460
   tttttggagg taatacctca aacaaacgct taaacaattt ttattggatt tttcttatag 5520
   gttttatatt tagagaaaaa agttcgaatt acggggtttg ttatgcaaaa taaaagcaaa 5580
   gtgagggacg attttattaa aattgttaaa gatgtgaaaa aagatttccc cgaattagac 5640
   ctaaaaatac gagtaaacaa ggaaaaagta actttcttaa attctccctt agaactctac 5700
   cataaaagtg tctcactaat tctaggactg cttcaacaaa tagaaaactc tttaggatta 5760
   ttcccagact ctcctgttct tgaaaaatta gaggataaca gtttaaagct aaaaaaggct 5820
   ttgattatgc ttatcttgtc tagaaaagac atgttttcca aggctgaata gataacttac 5880
   tctaacgttg gagttgattt gcacacctta gttttttgct cttttaaggg aggaactgga 5940
   aaaacaacac tttctctaaa cgtgggatgc aacttggccc aatttttagg gaaaaaagtg 6000
   ttacttgctg acctagaccc gcaatccaat ttatcttctg gattgggggc tagtgtcaga 6060
   agtaaccaaa aaggcttaca cgacatagta tacacatcaa acgatttaaa atcaatcatt 6120
   tgcgaaacaa aaaaagatag tgtggaccta attcctgcat catttttatc cgaacagttt 6180
   agagaattgg atattcatag aggacctagt aacaacttaa agttatttct gaatgagtac 6240
   tgcgctcctt tttatgacat ctgcataata gacactccac ctagcctagg agggttaacg 6300
   aaagaagctt ttgttgcagg agacaaatta attgcttgtt taactccaga acctttttct 6360
   attctagggt tacaaaagat acgtgaattc ttaagttcgg tcggaaaacc tgaagaagaa 6420
   cacattcttg gaatagcttt gtctttttgg gatgatcgta actcgactaa ccaaatgtat 6480
   atagacatta tcgagtctat ttacaaaaac aagctttttt caacaaaaat tcgtcgagat 6540
   atttctctca gccgttctct tcttaaagaa gattctgtag ctaatgtcta tccaaattct 6600
   agggccgcag aagatattct gaagttaacg catgaaatag caaatatttt gcatatcgaa 6660
   tatgaacgag attactctca gaggacaacg tgaacaaact aaaaaaagaa gcgaatgtct 6720
   tttttaaaaa aaatcaaact gccgcttctt tagattttaa gaagacgctt ccttccattg 6780
   aactattctc agcaactttg aattctgagg aaagtcagag tttggatcaa ttatttttat 6840
   cagagtccca aaactattcg gatgaagaat tttatcaaga agacatccta gcggtaaaac 6900
   tgcttactgg tcagataaaa tccatacaga agcaacacgt acttctttta ggagaaaaaa 6960
   tctataatgc tagaaaaatc ctgagtaagg atcacttctc ctcaacaact ttttcatctt 7020
   ggatagagtt agtttttaga actaagtctt ctgcttacaa tgctcttgca tattacgagc 7080
   tttttataaa cctccccaac caaactctac aaaaagagtt tcaatcgatc ccctataaat 7140
   ccgcatatat tttggccgct agaaaaggcg atttaaaaac caaggtcgat gtgataggga 7200
   aagtatgtgg aatgtcgaac tcatcggcga taagggtgtt ggatcaattt cttccttcat 7260
   ctagaaacaa agacgttaga gaaacgatag ataagtctga ttcagagaag aatcgccaat 7320
   tatctgattt cttaatagag atacttcgca tcatgtgttc cggagtttct ttgtcctcct 7380
   ataacgaaaa tcttctacaa cagctttttg aactttttaa gcaaaagagc tgatcctccg 7440
   tcagctcata tatatatcta ttatatatat atatttaggg atttgatttt acgagagaga 7500
<210> 2
   <211> 27
   <212> DNA
   <213> Artificial Sequence
<220>
   <223> Primer
<400> 2
   atcgcatgca agatatcgag tatgcgt 27
<210> 3
   <211> 27
   <212> DNA
   <213> Artificial Sequence
<220>
   <223> primer
<400> 3
   ctcataatta gcaagctgcc tcagaat 27
<210> 4
   <211> 26
   <212> DNA
   <213> Artificial Sequence
<220>
   <223> primer
<400> 4
   tgcctcctgt cccagtatct aaggtt 26
<210> 5
   <211> 24
   <212> DNA
   <213> Artificial Sequence
<220>
   <223> primer
<400> 5
   tgcaagtagt ctggatgttc ctgc 24
<210> 6
   <211> 27
   <212> DNA
   <213> Artificial Sequence
<220>
   <223> primer
<400> 6
   gcaaccagag acaactgatc tctactg 27
<210> 7
   <211> 26
   <212> DNA
   <213> Artificial sequence
<220>
   <223> primer
<400> 7
   ttctgcttgt ccagctggac catcta 26
<210> 8
   <211> 588
   <212> PRT
   <213> unknown
<220>
   <223> uncultured newly isolated virus
<400> 8
   Met Gly Glu Asp Gly Leu Ser Leu Pro Lys Met Met Asn Thr Pro Lys
   1               5                   10                  15
   Pro Ile Leu Lys Pro Gln Pro Lys Ala Leu Val Glu Pro Val Leu Cys
               20                  25                  30
   Asp Ser Ile Asp Glu Ile Pro Ala Lys Tyr Asn Glu Pro Val Tyr Phe
           35                  40                  45
   Asp Leu Glu Thr Asp Glu Asp Arg Pro Val Leu Ala Ser Ile Tyr Gln
       50                  55                  60
   Pro His Phe Glu Arg Lys Val Tyr Cys Leu Asn Leu Leu Lys Glu Lys
   65                  70                  75                  80
   Val Ala Arg Phe Lys Asp Trp Leu Leu Lys Phe Ser Glu Ile Arg Gly
                   85                  90                  95
   Trp Gly Leu Asp Phe Asp Leu Arg Val Leu Gly Tyr Thr Tyr Glu Gln
               100                 105                 110
   Leu Arg Asn Lys Lys Ile Val Asp Val Gln Leu Ala Ile Lys Val Gln
           115                 120                 125
   His Tyr Glu Arg Phe Lys Gln Gly Gly Thr Lys Gly Glu Gly Phe Arg
       130                 135                 140
   Leu Asp Asp Val Ala Arg Asp Leu Leu Gly Ile Glu Tyr Pro Met Asn
   145                 150                 155                 160
   Lys Thr Lys Ile Arg Glu Thr Phe Lys Asn Asn Met Phe His Ser Phe
                   165                 170                 175
   Ser Asn Glu Gln Leu Leu Tyr Ala Ser Leu Asp Ala Tyr Ile Pro His
               180                 185                 190
   Leu Leu Tyr Glu Gln Leu Thr Ser Ser Thr Leu Asn Ser Leu Val Tyr
           195                 200                 205
   Gln Leu Asp Gln Gln Ala Gln Lys Val Val Ile Glu Thr Ser Gln His
   210 215 220
   Gly Met Pro Val Lys Leu Lys Ala Leu Glu Glu Glu Ile His Arg Leu
   225 230 235 240
   Thr Gln Leu Arg Ser Glu Met Gln Lys Gln Ile Pro Phe Asn Tyr Asn
   245 250 255
   Ser Pro Lys Gln Thr Ala Lys Phe Phe Gly Val Asn Ser Ser Ser Lys
   260 265 270
   Asp Val Leu Met Asp Leu Ala Leu Gln Gly Asn Glu Met Ala Lys Lys
   275 280 285
   Val Leu Glu Ala Arg Gln Ile Glu Lys Ser Leu Ala Phe Ala Lys Asp
   290 295 300
   Leu Tyr Asp Ile Ala Lys Arg Ser Gly Gly Arg Ile Tyr Gly Asn Phe
   305 310 315 320
   Phe Thr Thr Thr Ala Pro Ser Gly Arg Met Ser Cys Ser Asp Ile Asn
   325 330 335
   Leu Gln Gln Ile Pro Arg Arg Leu Arg Ser Phe Ile Gly Phe Asp Thr
   340 345 350
   Glu Asp Lys Lys Leu Ile Thr Ala Asp Phe Pro Gln Ile Glu Leu Arg
   355 360 365
   Leu Ala Gly Val Ile Trp Asn Glu Pro Lys Phe Ile Glu Ala Phe Arg
   370 375 380
   Gln Gly Ile Asp Leu His Lys Leu Thr Ala Ser Ile Leu Phe Asp Lys
   385 390 395 400
   Asn Ile Glu Glu Val Ser Lys Glu Glu Arg Gln Ile Gly Lys Ser Ala
   405 410 415
   Asn Phe Gly Leu Ile Tyr Gly Ile Ala Pro Lys Gly Phe Ala Glu Tyr
   420 425 430
   Cys Ile Ala Asn Gly Ile Asn Met Thr Glu Glu Gln Ala Tyr Glu Ile
   435 440 445
   Ser Gln Lys Val Glu Glu Val Leu His Lys Asp Cys Arg Gln His Gln
   450 455 460
   Val Ala Tyr Glu Arg Phe Lys Tyr Asn Glu Tyr Val Asp Asn Glu Thr
   465 470 475 480
   Trp Leu Asn Arg Thr Tyr Arg Ala Trp Lys Pro Gln Asp Leu Leu Asn
                   485                 490                 495
   Tyr Gln Ile Gln Gly Ser Gly Ala Glu Leu Phe Lys Lys Ala Ile Val
               500                 505                 510
   Leu Leu Lys Glu Thr Lys Pro Asp Leu Lys Ile Val Asn Leu Val His
           515                 520                 525
   Asp Glu Ile Val Val Glu Ala Asp Ser Lys Glu Ala Gln Asp Leu Ala
       530                 535                 540
   Lys Leu Ile Lys Glu Lys Met Glu Glu Ala Trp Asp Trp Cys Leu Glu
   545                 550                 555                 560
   Lys Ala Glu Glu Phe Gly Asn Arg Val Ala Lys Ile Lys Leu Glu Val
                   565                 570                 575
   Glu Glu Pro His Val Gly Asn Thr Trp Glu Lys Pro
               580                 585

## Description

### BACKGROUND

Amplification of nucleic acids is widely used in research, forensics, medicine and agriculture. Polymerase chain reaction (PCR) is the most widely used method for *in vitro* DNA amplification. A PCR reaction typically utilizes two oligonucleotide primers that are hybridized to the 5' and 3' borders of the target sequence and a DNA-dependant DNA polymerase that extends the annealed primers by polymerizing deoxyribonucleotide-triphosphates (dNTPs) to generate double-stranded products. By raising and lowering the temperature of the reaction mixture (known as thermocycling), the two strands of the DNA product are separated and can serve as templates for the next round of annealing and extension, and the process is repeated.

In the past several years, other nucleic acid amplification methods have been developed that do not rely on thermocycling. These methods are broadly categorized as "isothermal target amplifications," owing to the fact that they do not rely on repeated cycles of temperature change to operate.

One such example is helicase-dependent amplification (HDA). *In vivo,* polymerases amplify nucleic acids with the aid of a variety of accessory proteins. One such class of accessory proteins is termed "helicases," which share the common characteristic of separating duplexed strands of nucleic acids into single strands, which are then accessible to polymerases for amplification. HDA mimics this general scheme *in vitro* by utilizing a helicase to generate single-stranded templates for primer hybridization and subsequent primer extension by a polymerase. By adding the helicase to the reaction mixture, repeated rounds of amplification can proceed without a need to repeatedly melt and re-anneal the primers to the templates. Accordingly, expensive thermocycling devices or tedious manual thermocycling can be avoided. In addition, HDA offers several advantages over other isothermal DNA amplification methods by having a simple reaction scheme and being a true isothermal reaction that can be performed at one temperature for the entire process.

One variation of PCR - termed reverse transcriptase PCR (RT-PCR) - is frequently used to measure gene expression, analyze RNA in samples, and synthesize modified complementary cDNA probes, among other uses. In the typical scheme, an enzyme having reverse transcriptase activity uses an RNA template to generate a complementary DNA strand (cDNA), which is then amplified via PCR. HDA may also be used to amplify the cDNA, in which case the process is termed reverse transcriptase HDA (RT-HDA). In either case, separate enzymes typically are used for each activity: a reverse transcriptase for generating a cDNA; a DNA-dependant DNA polymerase for amplifying the cDNA; and, in the case of HDA, a helicase for generating single stranded templates.

Unfortunately, reverse transcriptase enzymes can be highly error-prone, as they typically do not possess proof-reading abilities. Further, each enzyme added to the reaction mixture increases the potential necessity for different optimal temperatures, reaction conditions, reagents, etc. Thus, finding a set of enzymes and a set of conditions which produce high amounts of high fidelity DNA is often a difficult task One way to simplify this task is to reduce the number of enzymes involved.
WO 2010/088273 describes methods of amplifying a target nucleic acid in a helicase-dependent reaction. As DNA polymerase that can be used inter alia a polymerase capable of carrying out the reverse transcription reaction as well as DNA polymerase activity in the tHDA reaction can be used. WO 2006/074336 describes a method for identifying an RNA molecule that includes (a) synthesizing a cDNA from the RNA molecule by reverse transcription to form an RNA/DNA duplex; (b) at least partially separating the duplex into a cDNA and an RNA using a thermostable helicase; (c) amplifying the cDNA; and (d) characterizing the cDNA to determine the identity of the RNA molecule. Reverse transcription is achieved by means of a reverse transcriptase or a DNA polymerase with reverse transcription activity. WO 2007/044671 describes thermostable viral polymerases and inter alia describes the sequence of PYROPHAGE 3173.
WO 2007/120808 discloses a helicase-dependent asymmetric amplification (HAD) method, wherein UvrD helicase and Bst DNA polymerase is used to obtain the amplified product.
WO 2005/095654 discloses a circular-helicase-dependent amplification (cHDA) method for isothermal amplifying nucleic acids from a cDNA template. The system combines a DNA polymerase and a helicase preparation to amplify a target sequence as well as the entire circular DNA template containing the target sequence. WO 2004/027025 describes a helicase-dependent amplification of nucleic acids. In said method a helicase preparation and a DNA polymerase is used such that the amplification can be performed isothermally. Motré et al, Gene 420:17-22, 2008 discloses the enhancing of a helicase-dependent amplification using a fusion protein, which was prepared by fusing the helicase with the DNA polymerase; "helimerase"-activity to obtain amplified nucleic acid. Goldmeyer et al, Journal of Molecular Diagnostics 9(5):639-644, 2007 discloses the development of a one-tube isothermal reverse transcription thermophilic helicase-dependent amplification platform for RNA detection. In the described method, cDNA copies were generated and amplified from an RNA target concurrently using a helicase, a reverse transcriptase and a DNA polymerase. Yong-Joo Jeong et al, CMLS Cellular and Molecular Life Sciences 66(20):3325-3336, 2009 discloses the isothermal DNA amplification in vitro using a helicase-dependent amplification system to obtain amplified nucleic acids. Lixin An et al; J Biol Chem. 280(32):28952-28958, 2005 describes the characterization of a thermostable UvrD helicase and its participation in helicase-dependent nucleic acid amplification.

### SUMMARY

Disclosed herein are materials and methods for performing an isothermal amplification of a target nucleic acid using an enzyme having both reverse transcriptase activity and DNA-dependant DNA polymerase activity.

One aspect is directed to a method for isothermal amplification of a target nucleic acid using a first enzyme having helicase activity and a second enzyme having both reverse transcriptase and DNA-dependant DNA polymerase activities wherein the second enzyme is PYROPHAGE 3173.

Another aspect is directed to a method for isothermal amplification of a target RNA using a first enzyme having helicase activity and a second enzyme having both reverse transcriptase and DNA-dependant DNA polymerase activities wherein the second enzyme is PYROPHAGE 3173.

Another aspect is directed to a method of isothermal amplification of a target RNA using a first enzyme having nick-inducing activity and a second enzyme having both reverse transcriptase and DNA-dependant DNA polymerase activities wherein the second enzyme is PYROPHAGE 3173.

Another aspect is directed to a method of RT-HDA using a first enzyme having helicase activity and a second enzyme having both reverse transcriptase activity and DNA-dependant DNA polymerase activity wherein the second enzyme is PYROPHAGE 3173.

Another aspect is directed to a method of identifying the presence of a human papilloma virus (HPV) in a sample comprising detecting a nucleic acid sequence of the HPV by using a first enzyme having a helicase activity and a second enzyme having both reverse transcriptase activity and DNA-dependant DNA polymerase activity wherein the second enzyme is PYROPHAGE 3173.

Yet another aspect is directed a kit for isothermal amplification of a target nucleic acid comprising an first enzyme having a helicase activity and a second enzyme having both reverse transcriptase activity and DNA-dependant DNA polymerase activity wherein the second enzyme is PYROPHAGE 3173.

In another aspect, a kit for isothermal amplification of a target RNA is provided comprising an enzyme having helicase activity and an enzyme having both reverse transcriptase activity and DNA-dependant DNA polymerase activity wherein said enzyme is PYROPHAGE 3173 and does not comprise any other enzymes having DNA-dependant DNA polymerase activity.

Another aspect is directed to a kit for RT-HDA in which an enzyme having reverse transcriptase activity or an enzyme having polymerase activity, or both, are replaced by an enzyme having reverse transcriptase activity and DNA-dependant DNA polymerase activity wherein said enzyme is PYROPHAGE 3173.

Another aspect is directed to a kit for determining the presence and/or abundance of at least one human papilloma virus (HPV) in a sample comprising a first enzyme having helicase activity and a second enzyme having both reverse transcriptase activity and DNA-dependant DNA polymerase activity wherein the second enzyme is PYROPHAGE 3173.

### BRIEF DESCRIPTION OF THE FIGURES

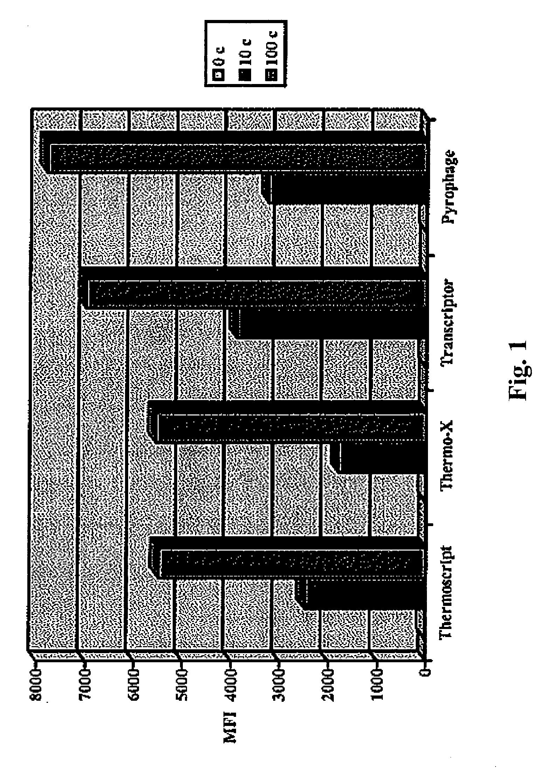

FIG. 1 shows that PYROPHAGE 3173 is as effective as other reverse transcriptases in one-step, RT-HDA in the presence of Bst-polymerase. Amplification was performed in 25 µl for 75 minutes, utilizing Bst polymerase (2U) and uvrD helicase (1U). CtRNA was used as a target. The assay signal (Luminex MFI) for Thermoscript, Thermo-X, Transcriptor and PYROPHAGE is given for 10 and 100 RNA copies.

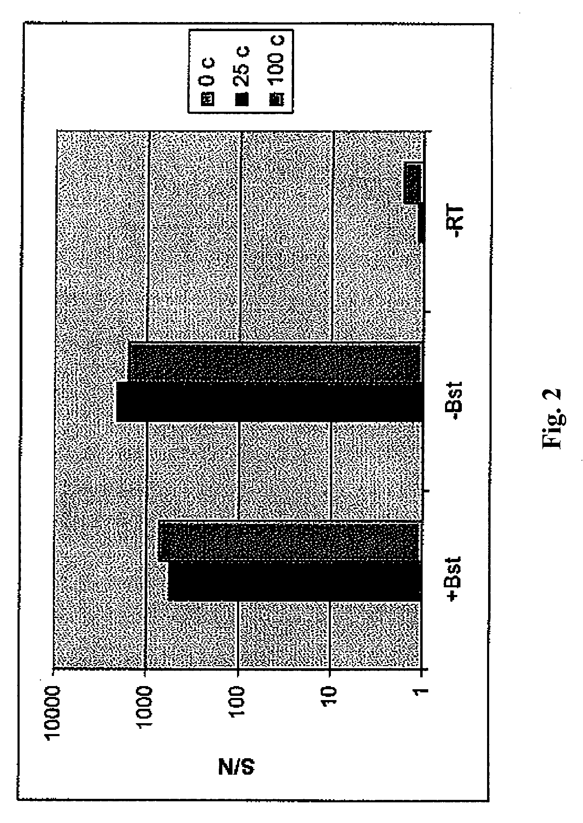

FIG. 2 shows that PYROPHAGE 3173 enzyme could perform as both a reverse transcriptase and a DNA-dependant DNA polymerase in a one step isothermal RT-HDA. Amplification was performed in 25 µL for 75 minute. The reaction mixture contained PYROPHAGE 3173 (2.5 U) either with Bst (bars labeled Bst +) or without the addition of Bst polymerase (bars labeled Bst-). Ct RNA (25 or 100 copies) was used as a target. Detection was performed on a Luminex and the results are presented as Signal/Noise.

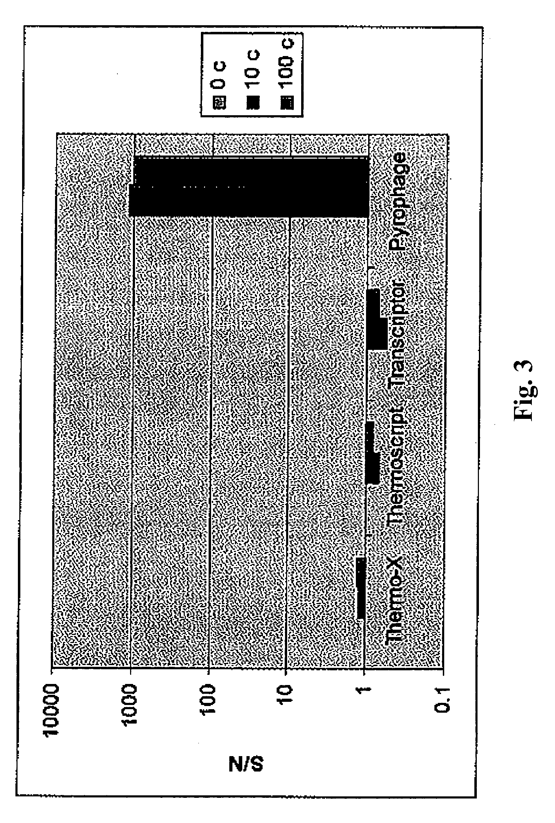

FIG. 3 shows RT-HDA reactions using THERMO-X, THERMOSCRIPT, TRANSCRIPTOR, and PYROPHAGE 3173, in the absence of Bst-polymerase. Reaction conditions are the same as described in Figure 2.

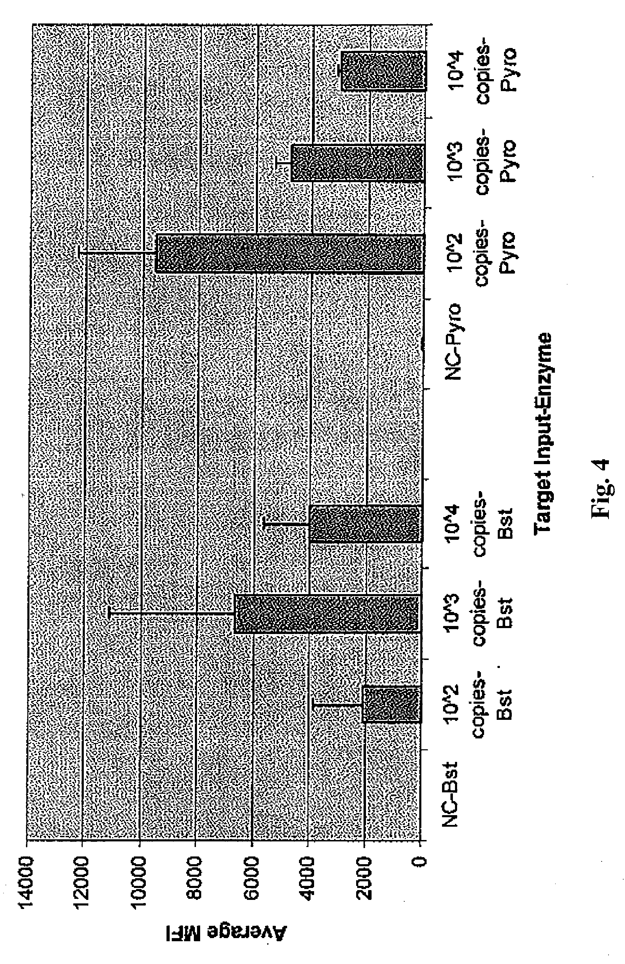

FIG. 4 shows that PYROPHAGE 3173 enzyme could amplify DNA in the absence of Bst. Amplification was performed in 50 µL for 90 minute. Reactions contained either PYROPHAGE 3173 (5U) or Bst-polymerase (20U). HPV16 DNA was used as a target for standard tHDA assay with alkaline denaturation. Detection was performed on a Luminex and the results are presented as a Signal/Noise.

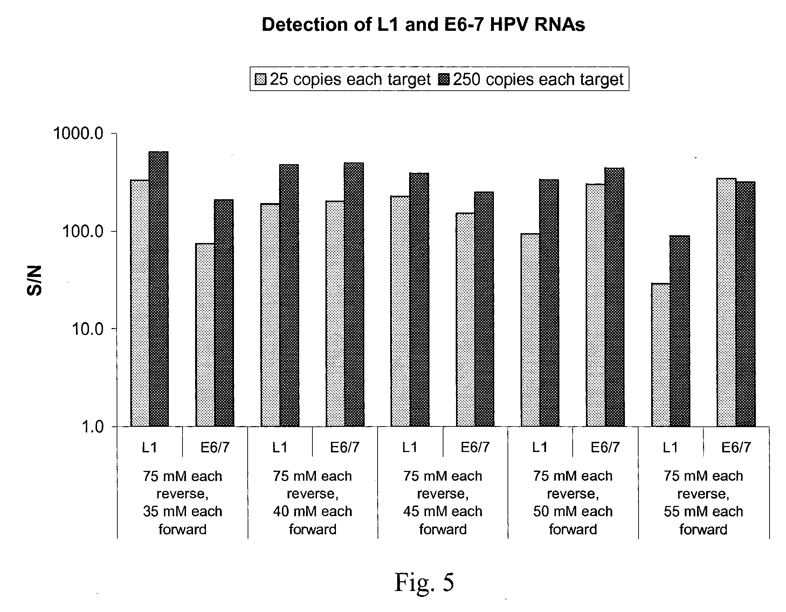

FIG. 5 shows the signal over noise (S/N) for RT-HDA reactions in which two HPV 16 RNA targets are amplified. Each bar represents S/N (y-axis) for each of the two targets after amplification with various primer concentrations. Primer concentrations and the amplified targets are indicated on the x-axis.

### DETAILED DESCRIPTION

In one aspect, amplification of a target nucleic acid is accomplished by an enzyme having both reverse transcriptase activity and DNA-dependant DNA polymerase activity wherein said enzyme is PYROPHAGE 3173. This enzyme, with dual activities, is used as a substitute for, or in addition to, using a DNA-dependant DNA polymerase and/or a reverse transcriptase.

As used herein, "nucleic acid" refers to double stranded (ds) or single stranded (ss) DNA, RNA molecules or DNA-RNA hybrids. Double stranded nucleic acid molecules may be nicked or intact. The double stranded or single stranded nucleic acid molecules may be linear or circular. The duplexes may be blunt ended or have single stranded tails. The single stranded molecules may have secondary structure in the form of hairpins or loops and stems. The nucleic acid may be isolated from a variety of sources including the environment, food, agriculture, fermentations, biological fluids such as blood, milk, cerebrospinal fluid, sputum, saliva, stool, lung aspirates, swabs of mucosal tissues or tissue samples or cells. Nucleic acid samples may be obtained from cells, bacteria or viruses and may include any of: chromosomal DNA, extra chromosomal DNA including plasmid DNA, recombinant DNA, DNA fragments, messenger RNA, transfer RNA, ribosomal RNA, double stranded RNA or other RNAs that occur in cells, bacteria or viruses. The nucleic acid may be isolated, cloned or synthesized *in vitro* by means of chemical synthesis. Any of the above described nucleic acids may be subject to modification where individual nucleotides within the nucleic acid are chemically altered (for example, by methylation). Modifications may arise naturally or by *in vitro* synthesis. The term "duplex" refers to a nucleic acid molecule that is double stranded in whole or part.

As used herein, the term "target nucleic acid" refers to any nucleic acid sequence that is intended to be amplified. The size of the target nucleic acid to be amplified may be, for example, in the range of about 50 bp to about 100 kb including a range of above 100 to 5000 bp. The target nucleic acid may be contained within a longer double stranded or single stranded nucleic acid. Alternatively, the target nucleic acid may be an entire double stranded or single stranded nucleic acid.

The enzyme having both reverse transcriptase and DNA-dependant DNA polymerase activities is PYROPHAGE 3173. PYROPHAGE 3173 is described in U.S. Patent Application No. 12/089,221, published as U.S. Patent Application Publication No. 2008/02684980. PYROPHAGE 3173 as described in US 2008/0268498 has the following amino acid sequence: PYROPHAGE 3173, is available from LUCIGEN Corporation, and is a thermostable bacteriophage enzyme that has an inherent 3'→5' exonuclease (proofreading) activity, which results in high fidelity amplification. Because of this activity, it may be preferable to use phosphorothioate primers and minimal exposure of the target nucleic acid template and the primers prior to amplification. Alternatively, a mutant version may be used, in which the 3'→5' exonuclease activity has been inactivated (PYROPHAGE 3173 Exo- mutant). PYROPHAGE 3173 also has strand-displacing activity that allows for DNA synthesis through double-stranded DNA. It also initiates efficiently at nicks and therefore DNA synthesis can be initiated either with primers or at a nick introduced by site-specific nicking enzymes. PYROPHAGE 3173 also has reverse transcription activity and thus can perform single-tube, single enzyme reverse transcription PCR on RNA templates. Because of this dual activity PYROPHAGE 3173 may be used in RT-HDA as a substitute for reverse transcriptase and DNA-dependant DNA polymerase. In addition, the higher thermostability of PYROPHAGE 3173 enzyme may allow higher RNA amplification rate.

In one embodiment, the target nucleic acid is amplified using an isothermal amplification. "Isothermal amplification" refers to amplification which occurs at a single temperature. This does not include the single brief time period (less than 15 minutes) at the initiation of amplification, which may be conducted at the same temperature as the amplification procedure or at a higher temperature.

In one embodiment, the isothermal amplification method is RT-HDA. Traditionally, three enzymes are used in RT-HDA: a reverse transcriptase, a helicase, and a DNA-dependant DNA polymerase. Reverse transcriptase (also known as RNA-dependent DNA polymerase), is an enzyme having a DNA polymerase activity that transcribes single stranded RNA (ssRNA) into a complementary single stranded DNA (cDNA) by polymerizing deoxyribonucleotide triphosphates (dNTPs). The same pyrophage enzyme may also polymerize the "second strand" of the cDNA making ds-DNA. This negates the use of two enzymes (reverse transcriptase and DNA-dependant DNA polymerase) in the traditional process of ds-DNA synthesis from ss-RNA. The helicase has an enzymatic activity that unwinds the ds-DNA for iterations (amplification) of primer-dependant DNA polymerization of top and bottom strands of ds-DNA. The DNA-dependant DNA polymerase then transcribes the cDNA into a complementary single stranded DNA by polymerizing dNTPs. This process repeats itself so that exponential amplification can be achieved at a single temperature without necessitating thermocycling. RT-HDA is performed using a single enzyme to provide both the reverse transcriptase and DNA-dependant DNA polymerase activity.

As used herein, "HDA" refers to Helicase Dependent Amplification which is an *in vitro* method for amplifying nucleic acids by using a helicase preparation for unwinding a double stranded nucleic acid to generate templates for amplification.

As used herein, "Helicase" or "an enzyme with, or having, helicase activity" refers to any enzyme capable of unwinding a double stranded nucleic acid. For example, helicases are enzymes that are found in all organisms and in all processes that involve nucleic acid such as replication, recombination, repair, transcription, translation and RNA splicing. Any helicase that translocates along DNA or RNA in a 5'→3' direction or in the opposite 3'→5' direction may be used. This includes helicases obtained from prokaryotes, viruses, archaea, and eukaryotes or recombinant forms of naturally occurring enzymes as well as analogues or derivatives having the specified activity. Examples of naturally occurring DNA helicases include E. *coli* helicase I, II, III, & IV, Rep, DnaB, PriA, PcrA, T4 Gp41 helicase, T4 Dda helicase, T7 Gp4 helicases, SV40 Large T antigen, yeast RAD. Additional helicases that may be useful include RecQ helicase, thermostable UvrD helicases from *T. tengcongensis* and *T. thermophilus,* thermostable DnaB helicase from *T. aquaticus,* and MCM helicase from archaeal and eukaryotic organisms.

In another embodiment, the helicase is a thermostable helicase. Denaturation of nucleic acid duplexes can be accelerated by using a thermostable helicase preparation under incubation conditions that include higher temperature for example in a range of 45°C to 75°C. Performing HDA at high temperature using a thermostable helicase preparation and a thermostable polymerase may increase the specificity of primer binding, which can improve the specificity of amplification.

In a further embodiment, a plurality of different helicase enzymes is used in the amplification reaction. The use of a plurality of helicases may enhance the yield and length of target amplification in HDA under certain conditions where different helicases coordinate various functions to increase the efficiency of the unwinding of duplex nucleic acids. For example, a helicase that has low processivity but is able to melt blunt-ended DNA may be combined with a second helicase that has great processivity but recognizes single-stranded tails at the border of duplex region for the initiation of unwinding. In this example, the first helicase initially separates the blunt ends of a long nucleic acid duplex generating 5' and 3' single-stranded tails and then dissociates from that substrate due to its limited processivity. This partially unwound substrate is subsequently recognized by the second helicase that then continues the unwinding process with superior processivity. In this way, a long target in a nucleic acid duplex may be unwound by the use of a helicase preparation containing a plurality of helicases and subsequently amplified in a HDA reaction.

In a further embodiment, an accessory protein is included with the reaction mixture. "Accessory protein" refers to any protein capable of stimulating helicase activity. For example, E. *coli* MutL protein is an accessory protein for enhancing UvrD helicase activity. Accessory proteins are useful with selected helicases. However, unwinding of nucleic acids may be achieved by helicases in the absence of accessory proteins.

In another embodiment, at least one single-strand binding proteins (SSB) is included with the reaction mixture. Mesophilic helicases show improved activity in the presence ofSSBs. In these circumstances, the choice of SSB is generally not limited to a specific protein. Examples of single strand binding proteins are T4 gene 32 protein, E. coli SSB, T7 gp2.5 SSB, phage phi29 SSB and truncated forms of these proteins. Thus, in certain embodiments, one or more SSBs may be added to an amplification reaction.

In yet another embodiment, at least one cofactor is provided. "Cofactors" refer to small-molecule agents that are required for the helicase unwinding activity. Helicase cofactors include nucleoside triphosphate (NTP) and deoxynucleoside triphosphate (dNTP) and magnesium (or other divalent cations). For example, ATP (adenosine triphosphate) may be used as a cofactor for UvrD helicase at a concentration in the range of 0.1 to 100 mM and preferably in the range of 1 to 10 mM (for example 3 mM). Similarly, dTTP (deoxythymidine triphosphate) may be used as a cofactor for T7 Gp4B helicase in the range of 1 to 10 mM (for example 3 mM).

In a further embodiment, the DNA-dependant DNA polymerase transcribes the cDNAs in a sequence-dependent amplification. "Sequence-dependent synthesis" or "sequence-dependent amplification" refers to amplification of a target sequence relative to non-target sequences present in a sample with the use of target-specific primers. As used herein, "target-specific primer" refers to a single stranded nucleic acid capable of binding to a pre-determined single stranded region on a target nucleic acid to facilitate polymerase dependent replication of the target nucleic acid to be selectively amplified.

In one embodiment, a pair of target-specific primers, one hybridizing to the 5'-flank of the target sequence and the other hybridizing to the 3'-flank of the target, are used to achieve exponential amplification of a target sequence.

In another embodiment, multiple pairs of target-specific primers can be utilized in a single reaction for amplifying multiple targets simultaneously using different detection tags in a multiplex reaction. Multiplexing is commonly used in single nucleotide polymorphism (SNP) analysis and in detecting pathogens.

Generally, suitable target-specific primer pairs are short synthetic oligonucleotides, for example having a length of 10 or more nucleotides and less than 50 nucleotides. Target-specific, oligonucleotide primer design involves various parameters such as string-based alignment scores, melting temperature, primer length and GC content. When designing a target-specific primer, one of the important factors is to choose a sequence within the target fragment that is specific to the nucleic acid molecule to be amplified. Another important factor is to calculate the melting temperature of a target-specific primer for the reaction. The melting temperature of a target-specific primer is determined by the length and GC content of that oligonucleotide. Preferably the melting temperature of a primer is about 10 to 30°C higher than the temperature at which primer hybridization and target amplification will take place.

"Primer hybridization" refers to binding of an oligonucleotide primer to a region of the single-stranded nucleic acid template under the conditions in which the primer binds only specifically to its complementary sequence on one of the template strands, not other regions in the template. The specificity of hybridization may be influenced by the length of the oligonucleotide primer, the temperature in which the hybridization reaction is performed, the ionic strength, and the pH of the reaction mixture.

Each target-specific primer hybridizes to each end of the target nucleic acid and may be extended in a 3'→5' direction by a polymerase using the target nucleotide sequence as a template. To achieve specific amplification, a homologous or perfect match target-specific primer is preferred. However, target-specific primers may include sequences at the 5' end which are non-complementary to the target nucleotide sequence(s). Alternatively, target-specific primers may contain nucleotides or sequences throughout that are not exactly complementary to the target nucleic acid.

The target-specific primers may include any of the deoxyribonucleotide bases A, T, G or C and/or one or more ribonucleotide bases, A, C, U, G and/or one or more modified nucleotide (deoxyribonucleotide or ribonucleotide) wherein the modification does not prevent hybridization of the primer to the nucleic acid or elongation of the target-specific primer or denaturation of double stranded molecules. Target-specific primers may be modified with chemical groups such as phosphorothioates or methylphosphonates or with non nucleotide linkers to enhance their performance or to facilitate the characterization of amplification products.

In general, the temperature of denaturation suitable for permitting specificity of target-specific primer-template recognition and subsequent annealing may occur over a range of temperatures, for example 20°C to 75°C. A preferred denaturation temperature may be selected according to which helicase is selected for the melting process. Tests to determine optimum temperatures for amplification of a nucleic acid in the presence of a selected helicase can be determined by routine experimentation by varying the temperature of the reaction mixture and comparing amplification products using gel electrophoresis.

The target-specific primers may be subject to modification, such as fluorescent or chemiluminescent-labeling, and biotinylation (for example, fluorescent tags such as amine reactive fluorescein ester of carboxyfluorescein). Other labeling methods include radioactive isotopes, chromophores and ligands such as biotin or haptens, which while not directly detectable can be readily detected by reaction with labeled forms of their specific binding partners e.g. avidin and antibodies respectively. Such modifications can be used to detect the amplified products.

"Melting", "unwinding", or "denaturing" refer to separating all or part of two complementary strands of a nucleic acid duplex.

In a further embodiment, the DNA-dependant DNA polymerase transcribes the cDNA in a sequence-independent amplification. As used herein, "sequence-independent amplification" refers to any amplification performed by a DNA-dependant DNA polymerase that does not amplify a specific sequence. By way of example and not limitation, random primer mixtures or nick-inducing agents may be used to initiate sequence-independent amplification.

As used herein, "random primer mixture" refers to mixtures of short randomly generated oligonucleotide sequences.

As used herein, "nick-initiated polymerase activity" refers to polymerase activity in the absence of exogenous primers, which is initiated by single-strand breaks in the template. Synthesis initiates at the single-strand break in the DNA, rather than at the terminus of an exogenous synthetic primer. With nick-initiated synthesis, removal of primers is unnecessary, reducing cost, handling time and potential for loss or degradation of the product. In addition, nick-initiated synthesis reduces false amplification signals caused by self-extension of primers. The nicks may be introduced at defined locations, by using enzymes that nick at a recognition sequence, or may be introduced randomly in a target polynucleotide. As used herein, "nick-inducing agent" refers to any enzymatic or chemical reagent or physical treatment that introduces breaks in the phosphodiester bond between two adjacent nucleotides in one strand of a double-stranded nucleic acid. Examples of nick-inducing enzymes include Bpu10 I, BstNB I, Alw I, BbvC I, BbvC I, Bsm I, BsrD, and E. *coli* endonuclease I. In one embodiment, at least one nick-inducing enzyme is included as a replacement for a helicase in a reaction mixture. In another embodiment, at least one nick-inducing enzyme is added to a reaction mixture in addition to at least one helicase.

Other amplification reaction components may, in appropriate circumstances, include buffers, biomolecules, salts, urea, dimethyl-sulfoxide (DMSO), polyethylene glycol (PEG), magnesium, topoisomerases, accessory proteins, denaturating agents, cofactors, or mixtures thereof. When primer-initiated amplification is desired, primers are added to the amplification reaction components.

Deoxyribonucleotide triphosphates dNTPs (i.e., dATP, dGTP, dCTP and dTTP), are added, which are used to build the new strand of DNA. ATP or TTP are added as an energy source. ATP or TTP is a commonly preferred energy source for highly processive helicases. On average one ATP molecule is consumed by a DNA helicases to unwind 1 to 4 base pairs. To amplify a longer target, more ATP may be consumed as compared to a shorter target. In these circumstances, it may be desirable to include a pyruvate kinase-based ATP regenerating system for use with the helicase. Thus, in certain embodiments, ATP or TTP or a combination or a pyruvate kinase-based ATP regenerating system may be added to the amplification reaction components.

Topoisomerases can be used in long HDA reactions to increase the ability of HDA to amplify long target amplicons. When a very long linear DNA duplex is separated by a helicase, the swivel (relaxing) function of a topoisomerase removes the twist and prevents over-winding. For example, E. *coli* topoisomerase I can be used to relax negatively supercoiled DNA by introducing a nick into one DNA strand. DNA gyrase (topoisomerase II) introduces a transient double-stranded break into DNA allowing DNA strands to pass through one another. Thus, in certain embodiments, a topoisomerase or a gyrase, or both may be added to the amplification reaction.

In a further embodiment, an amplified nucleic acid product may be detected by various methods including ethidium-bromide staining and detecting the amplified sequence by means of a label, such as, but not limited to: a radiolabel, a fluorescent-label, and an enzyme. For example HDA amplified products can be detected in real-time using fluorescent-labeled LUX Primers (Invitrogen Corporation, Carlsbad, Calif.), which are oligonucleotides designed with a fluorophore close to the 3' end in a hairpin structure. This configuration intrinsically renders fluorescence quenching capability without separate quenching moiety. When the primer becomes incorporated into double-stranded amplification product, the fluorophore is dequenched, resulting in a significant increase in fluorescent signal.

The present disclosure also encompasses a kit comprising an enzyme with helicase activity and an enzyme with both reverse transcriptase activity and DNA-dependant DNA polymerase activity wherein said enzyme is PYROPHAGE 3173. The kit may further comprise amplification reaction components selected from, but not limited to, one or more of dNTPs, ATP, TTP, primers, magnesium, topoisomerases, SSB proteins, accessory proteins, denaturating agents, polyethylene glycol, cofactors, or mixtures thereof.

A further embodiment relates to a mixture comprising a nucleic acid that is a target for isothermal amplification. The nucleic acid target may be ssDNA, dsDNA, ssRNA, dsRNA, RNA-DNA hybrid, or a mixture of any of the above. The mixture comprising the target nucleic acid also comprises at least one enzyme with helicase activity and at least one enzyme with both reverse transcriptase activity and DNA-dependant DNA polymerase activity wherein said enzyme is PYROPHAGE 3173. The mixture comprising the target nucleic acid and the enzymes can also comprise one or more of the amplification reaction components previously described.

Another aspect is an amplified nucleic acid obtained by the amplification methods described. The amplified nucleic acid may be DNA or RNA.

In another aspect, a kit is provided for detecting an HPV RNA using an isothermal reverse transcriptase/amplification reaction, wherein the kit comprises at least one enzyme having both reverse transcriptase and DNA-dependant DNA polymerase activity wherein said enzyme is PYROPHAGE 3173. In one embodiment, the kit further comprises at least one enzyme having an activity selected from the group consisting of helicase activity and nick-inducing activity. In another embodiment, the kit further comprises at least one enzyme having a helicase activity and at least one enzyme having a nick-inducing activity. The kit may further comprise other reagents necessary for conducting the desired amplification, including but not limited to: buffers; biomolecules; salts; urea; dimethyl-sulfoxide (DMSO); polyethylene glycol (PEG); magnesium; topoisomerase; gyrase; accessory proteins; denaturating agents; cofactors; dNTPs; ATP; TTP; sequence-specific primer sets, including but not limited to unlabelled primers and labeled primers, such as biotinylated primers and LUX Primers; and random primers.

As used, the term "comprising" includes "consisting essentially of" and "consisting of'.

### EXAMPLES

### Example 1: Use of PYROPHAGE 3173 as a replacement for RT enzymes in RT-HDA

PYROPHAGE 3173 DNA polymerase has several advantages over Transcriptor and Thermoscript reverse transcriptase. For example, it is known that Thermoscript and Transcriptor have limited activity at 65°C in the HDA buffer. PYROPHAGE 3173 DNA polymerase was tested to determine whether it could replace these reverse transcriptases in a one step isothermal RT-HDA amplification.

Briefly, 25 µL reaction mixtures were created comprising: (1) 0, 10, or 100 copies of an in vitro transcribed, synthetic RNA comprising the Chlamydia trachomatis cryptic plasmid RNA (ct-RNA) (GenBank Accession number X06707) (SEQ ID NO: 1); (2) forward primer 5'-ATC GCA TGC AAG ATA TCG AGT ATG CGT-3' (SEQ ID NO: 2) and reverse primer 5'-CTC ATA ATT AGC AAG CTG CCT CAG AAT-3' ("ct-*orf* primers") (SEQ ID NO: 3); (3) 2.5 U of Thermoscript, Thermo-X, Transcriptor, or Pyrophage 3173; (4) 2U of Bst polymerase; and (5) 1 U of *uvrD* helicase. Amplications were performed at 65°C for 75 minutes. Reaction mixtures contained the final concentrations of reagents set forth in Table 1. As can be seen at Fig. 1, PYROPHAGE 3173 performs as well as amplification other RT enzymes.

**Table 1**

| **Reagent** | **Final Concentration** |
|---|---|
| Tris-HCl | 20 mM |
| KCl | 10 mM |
| MgSO₄ | 4 mM |
| NaCl | 40 mM |
| dNTP | 0.4 mM |
| dATP | 3 mM |

### Example 2: Use of PYROPHAGE 3173 as a replacement for both RT and DNA-dependant DNA polymerase

PYROPHAGE 3173 DNA polymerase was tested to determine whether it could replace both reverse transcriptase and DNA-dependant DNA polymerase in a one step isothermal RT-HDA amplification. Briefly, 25 µL reaction mixtures were created comprising: (1) 0, 25, or 100 copies of a ct-RNA; (2) ct-*orf* primers; (3) 2.5 U of Pyrophage 3173; (4) either 0U or 2U of Bst polymerase; and (5) 1 U of *uvrD* helicase. Reaction mixtures contained the final concentrations of reagents set forth in Table 1. Amplications were performed at 62°C or 65°C for 75 minutes. Results are shown at Fig. 2. As can be seen, PYROPHAGE 3173 is capable of replacing both reverse transcriptase and DNA-dependant DNA polymerase in a one step isothermal RT-HDA.

PYROPHAGE 3173 DNA polymerase also was compared to other enzymes having reverse transcriptase activity for the ability to perform RT-HDA in the absence of a separate DNA-dependant DNA polymerase. Briefly, 25 µL reaction mixtures were created comprising: (1) 0, 25, or 100 copies of a ct-RNA; (2) ct-*orf* primers; (3) 2.5 U of Thermoscript, Thermo-X, Transcriptor, or Pyrophage 3173; and (4) 1 U of *uvrD* helicase. Amplifications were performed at 65°C for 75 minutes. Detection by Luminex as in Example 1. Results are shown at Fig. 3. In contrast to PYROPHAGE 3173, other reverse transcriptases are not effective substitutes for Bst-polymerase for RT-HDA. Little or no assay signal was observed for RT-HDA reactions utilizing Thermo-X, Thermoscript or Transcriptor, when Bst-polymerase was omitted. Only PYROPHAGE 3173 was able to generate signal in reaction which had no Bst-polymerase.

### Example 3: Use of PYROPHAGE 3173 for DNA amplification

*Target amplification.* HPV16 DNA was used as the target DNA in an HDA assay. The double stranded DNA target was denatured in 5 µl 0.1M NaOH at 65°C for 10 minutes. An equal volume of 0.2M Hepes was then added to neutralize the denatured target. 15 µl of premix and 25 µl of amplification mix were added to the target and incubated at 65°C for 1.5 hours. Premix and amplification mix constituents are set forth in Table 2.

*Amplicon detection.* The HDA product (5 µL) was transferred to a U-bottom hybridization plate and then diluted in 5 µl of 1X denaturation reagent (Digene HC2 DNR, Qiagen Gaithersburg, Inc., Gaithersburg, MD). The plate then was sealed and shaken for 30 seconds at 1100 rpm in a Digene shaker and incubated at room temperature for 15 minutes. A hybridization diluent (5 µl of 1X hc2 probe diluent, Qiagen Gaithersburg, Inc., Gaithersburg, MD) was added and the plate was resealed and shaken for 30 seconds at 1100 rpm in a Digene shaker. A Luminex Bead Cocktail (10 µl in 1X TE) having an oligonucleotide complementary to the amplicon was added to each well (3000 beads/well), the plate was sealed again and incubated at 50°C for 30 minutes with shaking in the dark. Strepavidin-Phycoerythrin (Moss Corp.) (10 µl diluted to 12.5ng/µl in PBS) was added and the plate again sealed and shaken for 5 minutes at 1100 rpm in a Digene shaker protected from light. Phosphate buffered saline (150 µl) was then added, the plate resealed and shaken for 1 minute at 800 rpm. Median fluorescence intensity (MFI) was determined using a Luminex 100 and Luminex 1.7 software. Results are shown at Fig. 4. MFI above a background level correlates with presence of the DNA target.

### Example 4: Detection of two HPV 16 mRNA sequences using one step RT-HDA

Synthetic, *in vitro* transcribed RNAs corresponding to the HPV 16 E6-7 gene and the HPV 16 L1 gene were used as targets. Either 25 or 250 copies of each target nucleic acid were included in each reaction. A one step isothermal RT-HDA reaction was run as in Example 2, using the primers set forth in Table 3 in place of the Ct-*orf* primers. The reverse primer was used at a final concentration of 75 mM, while the forward primer concentration was 35 mM, 40 mM, 45 mM, 50 mM, or 55 mM. Results are shown at Fig. 5. Detection of 25 copies each of the two HPV 16 RNAs was robust for the RT-HDA reactions. The optimal primer concentrations in this experiment was 75 mM each reverse, biotinylated primer and 40 or 45 mM of each forward primer. The coefficient of variation (n=3) for the S/N was low (12 to 22%) for these reactions.

**Table 2**

| | Reagents | Final Concentration | |
|---|---|---|---|
| **Premix** | 10X Annealing Buffer (100 mM KCl and 200 mM Tris HCl pH 8.8) | 1 | X |
| | Forward Primer | 50 | nM |
| | Reverse Primer | 75 | nM |
| **Amplification Mix** | Reagents | Final Concentration | |
| | 10X Annealing Buffer (100 mM KCl and 200 mM Tris HCl pH 8.8) | 1 | X |
| | MgSO₄ | 4 | mM |
| | NaCl | 40 | mM |
| | dNTP | 0.4 | mM |
| | dATP | 3 | mM |
| | DNA polymerase (Bst/Pyrophage) | (0.4/0.1) | U/µl |
| | Tte-UvrD helicase | 0.02 | U/µl |

**Table 3**

| | | |
|---|---|---|
| HPV16-L1 | Forward primer SEQ ID NO: 4 | 5' TGC CTC CTG TCC CAG TAT CTA AGG TT 3' |
| | Reverse primer SEQ ID NO: 5 | 5' Biotin-TGC AAG TAG TCT GGA TGT TCC TGC 3' |
| HPV16-E | Forward primer SEQ ID NO: 6 | 5' GCA ACC AGA GAC AAC TGA TCT CTA CTG 3' |
| | Reverse primer SEQ ID NO: 7 | 5' Biotin-TTC TGC TTG TCC AGC TGG ACC ATC TA 3' |

### Example 5: Use of PYROPHAGE 3173 in hybrid capture

***A. Hybrid capture technology***

Hybrid capture technology utilizes certain antibodies capable of bind to RNA:DNA hybrids in various methods of purifying and detecting specific target nucleic acids in a sample. Various iterations of the hybrid capture method are described in, *inter alia,* U.S. Patent Nos. 5,994,079, 6,027,897, 6,277,579, 6,686,151, and 7,439,016; US Patent Publication Nos. 2006/0051809 A1, 2009/0162851 A1, and 2009-0298187 A1; and PCT Publication No. WO 01/96608. The basic hybrid capture protocol comprises: (1) hybridizing a nucleic acid probe to the target nucleic acid to generate a DNA:RNA hybrid; (2) associating the DNA:RNA hybrid with a solid phase to facilitate isolation of the target nucleic acid; and (3) detecting the DNA:RNA hybrid. In various iterations, anti-DNA:RNA hybrid antibodies can be used in either step (2) or step (3). By way of example and not limitation, the anti-DNA:RNA hybrid antibody may be bound to the solid phase (covalently or otherwise), thereby mediating "capture" of the DNA:RNA hybrid to the solid phase. Alternatively, a nucleic acid probe bound to the solid phase (covalently or otherwise) may capture the DNA:RNA hybrid to the solid phase, which may then be detected by a detectably labeled anti-DNA:RNA hybrid antibody.

***B. Detection of a target isolated via hybrid capture***

As noted previously, hybrid capture utilizes DNA:RNA hybrids. Therefore, the identity of the desired target will be important. When the target nucleic acid molecule is DNA, the probe is preferably RNA and when the target nucleic acid is RNA, the probe is preferably DNA.

Sample comprising the target nucleic acid is collected in a tube and treated with a denaturation reagent, such as an alkaline solution, to render the target nucleic acid molecule accessible to hybridization. Additionally, alkaline treatment of protein effectively homogenizes the specimen to ensure reproducibility of analysis results for a given sample. It can also reduce the viscosity of the sample to increase kinetics, homogenize the sample, and reduce background by destroying any endogenous single stranded RNA nucleic acids, DNA-RNA hybrids or RNA-RNA hybrids in the sample. It also helps inactivate enzymes such as RNases and DNases that may be present in the sample. One skilled in that art would appreciate that if RNA is the target nucleic acid (as opposed to DNA), different reagents may be preferable including, but not limited to phenol extraction and TCA/acetone precipitation, and guanidinium thiocyanate-phenolchloroform extraction.

After the sample containing the nucleic acid is denatured, it is contacted with one or more polynucleotide probes under a condition sufficient for the one or more polynucleotide probes to hybridize to the target nucleic acid in the sample to form a double-stranded nucleic acid hybrid. The probe can be full length, truncated, or synthetic DNA or full length, truncated, or synthetic RNA. If the target nucleic acid is DNA, then the probe may be RNA and if the target nucleic acid is RNA, then the probe may be DNA. Preferably, the one or more polynucleotide probes are diluted in a probe diluent that also can act as a neutralizing hybridization buffer (to neutralize the basic denaturation reagent). The probe diluent used for DNA or RNA probes will differ due to the different requirements necessary for DNA versus RNA stability. For example, if the probes are RNA, it is preferable to neutralize the sample first and than add the probe or alternatively, add the RNA probe and neutralizing agent (probe diluent) to the sample at the same time as NaOH can destroy RNA. The probe diluent can be used to dissolve and dilute the probe and also help restore the sample to about a neutral pH, e.g., about pH 6 to about pH 9, to provide a more favorable environment for hybridization. Sufficient volume of probe diluent, preferably one-half volume of the sample, may be used to neutralize the base-treated sample.

After the probes are allowed to hybridize to the target nucleic acid molecule and to form a double-stranded nucleic acid hybrid, the hybrid is captured by an anti-hybrid antibody that is immobilized onto a paramagnetic beads. The hybrids are incubated with the anti-hybrid antibody at about 67°C to about 70°C for about 30 minutes. A magnetic field is then applied to the tube and the supernatant removed from the beads. The beads may then be washed with a suitable wash buffer comprising, for example, 40 mM Tris, pH 8.2, 100 mM NaCl, 0.5% Triton-X 100 and 0.05% sodium azide.

The captured nucleic acids may then be detected using any of the amplification schemes described herein.

***C. Increasing sensitivity of a hybrid capture assay***

In some iterations, amplification can be used to increase the sensitivity of hybrid capture assays, particularly when the target nucleic acid is expected to be present in low copy numbers. However, standard amplification techniques are not always compatible with the conditions in which hybrid capture may be used. For example, one particular application of hybrid capture technology is for screening assays in rural communities, where expensive thermocyclers and trained technicians are often unavailable. In such circumstances, it is beneficial to reduce the number of complicated reagents involved and simplify the steps, which often precludes use of standard PCR protocols. In such a circumstance, thermostable polymerases such as PYROPHAGE 3173 would be useful.

In one example, an amplification as described herein may be performed on a sample, with the resultant amplicons purified and detected by a hybrid capture assay as set forth in, for example, U.S. Patent Nos. 5,994,079, 6,027,897, 6,277,579, 6,686,151, and 7,439,016; US Patent Publication Nos. 2006/0051809 A1, 2009/0162851 A1, and 2009-0298187 A1; and PCT Publication No. WO 01/96608.

Alternatively, the target nucleic acid may be purified as set forth in Example 5B, then amplified as set forth herein. After separation, targets may be denatured, separated from the beads, and detected by a hybrid capture assay as set forth in, for example, U.S. Patent Nos. 5,994,079, 6,027,897, 6,277,579, 6,686,151, and 7,439,016; US Patent Publication Nos. 2006/0051809 A1, 2009/0162851 A1, and 2009-0298187 A1; and PCT Publication No. WO 01/96608.

***D. Adapting incompatible targets for use with available reagents***

As set forth above, hybrid capture is preferably used in combination with DNA:RNA hybrids. However, there may be instances where the target nucleic acid and the hybrid capture probes are both RNA. In such a case, it would be desirable to convert the target RNA to a DNA before performing hybrid capture. The reverse transcriptase activity of PYROPHAGE 3173 could be useful in such an embodiment.

RNA optionally may be extracted from the sample before performing the reverse transcription reaction, particularly if the desired target has a DNA equivalent likely to be present in the sample, such as when an mRNA is the desired target. Many methods of isolating total RNA and subsets thereof are well known in the art, including, for example, acid guanidinium thiocyanate-phenol-chloroform extraction and commercially available kits, such as the RNeasy® line of kits (Qiagen GmbH, Hilden, DE). Whether to isolate RNA before performing the reverse transcription reaction and the precise method of doing so will depend largely on the particular target and application and can be determined by a person of ordinary skill in the art.

Once the sample is prepared as desired, the reverse transcription reaction can be performed essentially as described herein. Where increased sensitivity is necessary or desired, a one step RT-HDA reaction may likewise be performed. Target isolation may then be performed by hybrid capture as described in Example 5B and/or target detection may be performed as described in, for example, U.S. Patent Nos. 5,994,079, 6,027,897, 6,277,579, 6,686,151, and 7,439,016; US Patent Publication Nos. 2006/0051809 A1, 2009/0162851 A1, and 2009-0298187 A1; and PCT Publication No. WO 01/96608.

## Claims

1. A method for isothermal amplification of a target nucleic acid, the method comprising reacting the target nucleic acid with a reaction mixture comprising:
a) a first enzyme having a helicase activity; and
b) a second enzyme having:
i. a reverse transcriptase activity; and
ii. a DNA-dependent DNA polymerase activity
wherein the second enzyme with reverse transcriptase activity is PYROPHAGE 3173.

2. The method of claim 1, having one or more of the following characteristics:
a) the target nucleic acid is selected from the group consisting of dsDNA, dsRNA, ssDNA, or ssRNA;
b) the target nucleic acid is an HPV nucleic acid;
c) wherein the target nucleic acid is a target RNA and said second enzyme converts the target RNA to a target DNA by a method comprising a reverse transcription reaction.

3. The method of claim 2 c) further comprising an amplification reaction wherein the second enzyme amplifies the target DNA.

4. The method of any of claims 1-3, having one or more of the following characteristics:
a) the reaction mixture further comprises a target specific nucleic acid primer or wherein the reaction mixture comprises a random primer;
b) the reaction mixture further comprises a topoisomerase or a gyrase;
c) the reaction mixture comprises:
a. KCl;
b. Tris HCl;
c. MgSO₄;
d. NaCl;
e. dNTP;
f. dATP; and
g. a primer set

5. The method of claim 4 wherein said primer set comprises a primer selected from the group consisting of SEQ ID NO: 2 through SEQ ID NO: 7.

6. The method of any of claims 1-5 further comprising isolating the target nucleic acid from a sample.

7. The method of claim 6 wherein the target nucleic acid is isolated from the sample by a method comprising:
a. generating a DNA:RNA hybrid comprising the target nucleic acid;
b. binding the DNA:RNA hybrid to a solid phase; and
c. separating the DNA:RNA hybrid bound to the solid phase from the sample.

8. The method of claim 7 wherein the DNA:RNA hybrid is bound to an anti-DNA:RNA antibody.

9. The method of claim 8 wherein the anti-DNA:RNA antibody is bound or adapted to be bound to the solid phase.

10. The method of claim 6 wherein the target nucleic acid is purified from the sample before the target nucleic acid is amplified or wherein the target nucleic acid is purified from the sample after the target nucleic acid is amplified.

11. A kit comprising
a) a first enzyme having a helicase activity; and
b) a second enzyme having:
i. a reverse transcriptase activity; and
ii. a DNA-dependent DNA polymerase activity,
wherein the second enzyme is PYROPHAGE 3173.

12. The kit of claim 11, further comprising at least one component selected from the group consisting of:
a. KCl;
b. Tris HCl;
c. MgSO₄;
d. NaCl;
e. dNTP;
f. dATP;
g. a gyrase
h. a topoisomerase;
i. a primer set;
j. a nucleic acid probe;
k. an anti-DNA:RNA hybrid antibody; and
l. a solid phase,
wherein each component optionally is a component of a stock solution.

13. The kit of claim 11 or claim 12 comprising a nucleic acid probe, an anti-DNA:RNA hybrid antibody, and a solid phase, wherein either:
a. the anti-DNA:RNA antibody is adapted to be bound to the solid phase or is bound to the solid phase; or
b. the nucleic acid probe is adapted to be bound to the solid phase or is bound to the solid phase.

14. A mixture comprising:
a. a target nucleic acid;
b. a first enzyme having a helicase activity; and
c. a second enzyme having:
i. a reverse transcriptase activity; and
ii. a DNA-dependent DNA polymerase activity,
wherein the second enzyme is PYROPHAGE 3173.

15. The mixture of claim 14, further comprising at least one component selected from the group consisting of:
a. KCl;
b. Tris HCl;
c. MgSO₄;
d. NaCl;
e. dNTP;
f. dATP;
g. a gyrase
h. a topoisomerase;
i. a primer set;
j. a nucleic acid probe;
k. an anti-DNA:RNA hybrid antibody; and
l. a solid phase.

16. The mixture of claim 14 or 15 comprising a nucleic acid probe, an anti-DNA:RNA hybrid antibody, and a solid phase, wherein either:
a. the anti-DNA:RNA antibody is adapted to be bound to the solid phase or is bound to the solid phase; or
b. the nucleic acid probe is adapted to be bound to the solid phase or is bound to the solid phase.

## Patentansprüche

1. Verfahren zur isothermalen Amplifikation einer Zielnukleinsäure, umfassend reagieren der Zielnukleinsäure mit einem Reaktionsansatz, der aufweist:
a) ein erstes Enzym mit einer Helikase-Aktivität; und
b) ein zweites Enzym mit:
i. einer Reverse-Transkriptase-Aktivität; und
ii. einer DNA-abhängigen DNA-Polymerase-Aktivität,
wobei das zweite Enzym mit Reverse-Transkriptase-Aktivität PYROPHAGE 3173 ist.

2. Das Verfahren nach Anspruch 1 mit einem oder mehreren der folgenden Charakteristika:
a) die Zielnukleinsäure ist ausgewählt aus der Gruppe bestehend aus dsDNA, dsRNA, ssDNA oder ssRNA;
b) bei der Zielnukleinsäure handelt es sich um eine HPV-Nukleinsäure;
c) wobei es sich bei der Zielnukleinsäure um eine Ziel-RNA handelt und das zweite Enzym die Ziel-RNA mittels einem eine reverse Transkriptionsreaktion umfassenden Verfahren in eine Ziel-DNA umwandelt.

3. Das Verfahren nach Anspruch 2 c), ferner umfassend eine Amplifikationsreaktion, wobei das zweite Enzym die Ziel-DNA amplifiziert.

4. Das Verfahren nach irgendeinem der Ansprüche 1-3 mit einem oder mehreren der folgenden Charakteristika:
a) der Reaktionsansatz umfasst ferner einen zielspezifischen Nukleinsäure-Primer, oder wobei der Reaktionsansatz einen Random-Primer umfasst;
b) der Reaktionsansatz umfasst ferner eine Topoisomerase oder eine Gyrase;
c) der Reaktionsansatz umfasst:
a. KC1;
b. Tris-HCl;
c. MgSO₄;
d. NaCl;
e. dNTP;
f. dATP; und
g. ein Primer-Set.

5. Das Verfahren nach Anspruch 4, wobei das Primer-Set einen aus der aus SEQ ID NO: 2 bis SEQ ID NO: 7 bestehenden Gruppe ausgewählten Primer umfasst.

6. Das Verfahren nach irgendeinem der Ansprüche 1-5, ferner umfassend isolieren der Zielnukleinsäure aus einer Probe.

7. Das Verfahren nach Anspruch 6, wobei die Zielnukleinsäure aus der Probe mit einem Verfahren isoliert wird, bei dem man:
a. ein die Zielnukleinsäure umfassendes DNA:RNA-Hybrid erzeugt;
b. das DNA:RNA-Hybrid an eine feste Phase bindet; und
c. das an die feste Phase gebundene DNA:RNA-Hybrid von der Probe trennt.

8. Das Verfahren nach Anspruch 7, wobei das DNA:RNA-Hybrid an einen Anti-DNA:RNA-Antikörper gebunden ist.

9. Das Verfahren nach Anspruch 8, wobei der Anti-DNA:RNA-Antikörper an die feste Phase gebunden oder zur Bindung an die feste Phase angepasst ist.

10. Das Verfahren nach Anspruch 6, wobei die Zielnukleinsäure vor ihrer Amplifikation aus der Probe aufgereinigt wird oder wobei die Zielnukleinsäure nach ihrer Amplifikation aus der Probe aufgereinigt wird.

11. Kit, aufweisend
a) ein erstes Enzym mit einer Helikase-Aktivität; und
b) ein zweites Enzym mit:
i. einer Reverse-Transkriptase-Aktivität; und
ii. einer DNA-abhängigen DNA-Polymerase-Aktivität,
wobei das zweite Enzym PYROPHAGE 3173 ist.

12. Das Kit nach Anspruch 11, ferner aufweisend wenigstens eine Komponente ausgewählt aus der folgenden Gruppe:
a. KCl;
b. Tris-HCl;
c. MgSO₄;
d. NaCl;
e. dNTP;
f. dATP;
g. eine Gyrase;
h. eine Topoisomerase;
i. ein Primer-Set;
j. eine Nukleinsäuresonde;
k. einen Anti-DNA:RNA-Hybrid-Antikörper; und
l. eine feste Phase,
wobei es sich bei den Komponenten jeweils gegebenenfalls um eine Komponente einer Stammlösung handelt.

13. Das Kit nach Anspruch 11 oder Anspruch 12, umfassend eine Nukleinsäuresonde, einen Anti-DNA:RNA-Hybrid-Antikörper und eine feste Phase, wobei entweder:
a. der Anti-DNA:RNA-Antikörper zur Bindung an die feste Phase angepasst oder an die feste Phase gebunden ist; oder
b. die Nukleinsäuresonde zur Bindung an die feste Phase angepasst oder an die feste Phase gebunden ist.

14. Mischung, umfassend:
a. eine Zielnukleinsäure;
b. ein erstes Enzym mit einer Helikase-Aktivität; und
c. ein zweites Enzym mit:
i. einer Reverse-Transkriptase-Aktivität; und
ii. einer DNA-abhängigen DNA-Polymerase-Aktivität,
wobei das zweite Enzym PYROPHAGE 3173 ist.

15. Die Mischung nach Anspruch 14, ferner umfassend wenigstens eine Komponente ausgewählt aus der folgenden Gruppe:
a. KC1;
b. Tris-HCl;
c. MgSO₄;
d. NaCl;
e. dNTP;
f. dATP;
g. eine Gyrase;
h. eine Topoisomerase;
i. ein Primer-Set;
j. eine Nukleinsäuresonde;
k. einen Anti-DNA:RNA-Hybrid-Antikörper; und
l. eine feste Phase.

16. Die Mischung nach Anspruch 14 oder 15, aufweisend eine Nukleinsäuresonde, einen Anti-DNA:RNA-Hybrid-Antikörper und eine feste Phase, wobei entweder:
a. der Anti-DNA:RNA-Antikörper zur Bindung an die feste Phase angepasst oder an die feste Phase gebunden ist;
oder
b. die Nukleinsäuresonde zur Bindung an die feste Phase angepasst oder an die feste Phase gebunden ist.

## Revendications

1. Procédé d'amplification isotherme d'un acide nucléique cible, le procédé comprenant la réaction de l'acide nucléique cible avec un mélange réactionnel comprenant:
a) une première enzyme ayant une activité d'hélicase; et
b) une seconde enzyme ayant:
i. une activité de transcriptase inverse; et
ii. une activité d'ADN polymérase ADN-dépendante,
où la seconde enzyme à l'activité de transcriptase inverse est PYROPHAGE 3173.

2. Procédé selon la revendication 1, présentant une ou plusieurs des caractéristiques suivantes:
a) un acide nucléique cible est sélectionné dans le groupe composé de l'ADNdb, de l'ARNdb, de l'ADNsb ou de l'ARNsb;
b) l'acide nucléique cible est un acide nucléique de PVH;
c) où l'acide nucléique cible est un ARN cible et ladite seconde enzyme convertit l'ARN cible en ADN cible par un procédé comprenant une réaction de transcription inverse.

3. Procédé selon la revendication 2c) comprenant en outre une réaction d'amplification dans laquelle la seconde enzyme amplifie l'ADN cible.

4. Procédé selon l'une quelconque des revendications 1 à 3, présentant une ou plusieurs des caractéristiques suivantes:
a) le mélange réactionnel comprend en outre une amorce d'acide nucléique spécifique de la cible ou le mélange réactionnel comprend une amorce aléatoire;
b) le mélange réactionnel comprend en outre une topo-isomérase ou une gyrase;
c) le mélange réactionnel comprend:
a. KCl;
b. Tris HCl;
c. MgSO₄;
d. NaCl;
e. dNTP;
f. dATP; et
g. un jeu d'amorces

5. Procédé selon la revendication 4, dans lequel ledit jeu d'amorces comprend une amorce sélectionnée dans le groupe composé de l'ID SEQ. n° 2 à l'ID SEQ. n° 7.

6. Procédé selon l'une quelconque des revendications 1 à 5, comprenant en outre l'isolement de l'acide nucléique cible depuis l'échantillon.

7. Procédé selon la revendication 6, dans lequel l'acide nucléique cible est isolé de l'échantillon par un procédé comprenant les étapes consistant à:
a. générer un hybride ADN:ARN comprenant l'acide nucléique cible;
b. lier l'hybride ADN:ARN à une phase solide; et
c. séparer de l'échantillon l'hybride ADN:ARN lié à la phase solide.

8. Procédé selon la revendication 7, dans lequel l'hybride ADN:ARN est lié à un anticorps anti-ADN:ARN.

9. Procédé selon la revendication 8, dans lequel l'anticorps anti-hybride ADN:ARN est lié à ou est adapté pour être lié à la phase solide.

10. Procédé selon la revendication 6, dans lequel l'acide nucléique cible est purifié depuis l'échantillon avant que l'acide nucléique cible soit amplifié, ou dans lequel l'acide nucléique cible est purifié depuis l'échantillon après que l'acide nucléique cible est amplifié.

11. Kit comprenant:
a) une première enzyme présentant une activité d'hélicase; et
b) une seconde enzyme ayant:
i. une activité de transcriptase inverse; et
ii. une activité d'ADN polymérase ADN-dépendante,
où la seconde enzyme est PYROPHAGE 3173.

12. Kit selon la revendication 11, comprenant en outre au moins un composant sélectionné dans le groupe composé de:
a. KCl;
b. Tris HCl;
c. MgSO₄;
d. NaCl;
e. dNTP;
f. dATP;
g. une gyrase;
h. une topo-isomérase;
i. un jeu d'amorces;
j. une sonde d'acide nucléique;
k. et un anticorps anti-hybride ADN:ARN; et
l. une phase solide,
où chaque composant est en option un composant d'une solution mère.

13. Kit selon la revendication 11 ou la revendication 12, comprenant une sonde d'acide nucléique, un anticorps anti-hybride ADN:ARN, et une phase solide, dans lequel:
a. l'anticorps anti-ADN:ARN est adapté pour être lié à la phase solide ou est lié à la phase solide; ou
b. la sonde d'acide nucléique est adaptée pour être liée à la phase solide ou est liée à la phase solide.

14. Mélange comprenant:
a. un acide nucléique cible;
b. une première enzyme ayant une activité d'hélicase; et
c. une seconde enzyme ayant:
i. une activité de transcriptase inverse; et
ii. une activité d'ADN polymérase ADN-dépendante,
où la seconde enzyme est PYROPHAGE 3173.

15. Mélange selon la revendication 14, comprenant en outre au moins un composant sélectionné dans le groupe composé de:
a. KCl;
b. Tris HCl;
c. MgSO₄;
d. NaCl;
e. dNTP;
f. dATP;
g. une gyrase;
h. une topo-isomérase;
i. un jeu d'amorces;
j. une sonde d'acide nucléique;
k. et un anticorps anti-hybride ADN:ARN; et
l. une phase solide.

16. Mélange de la revendication 14 ou 15, comprenant une sonde d'acide nucléique, un anticorps anti-hybride ADN:ARN, et une phase solide, où:
a. l'anticorps anti-ADN:ARN est adapté pour être lié à la phase solide ou est lié à la phase solide; ou
b. la sonde d'acide nucléique est adaptée pour être liée à la phase solide ou est liée à la phase solide.
